(19)

**Europäisches Patentamt**
**European Patent Office**
**Office européen des brevets**

(11) **EP 4 708 114 A1**

(12) **EUROPEAN PATENT APPLICATION**
published in accordance with Art. 153(4) EPC

(43) Date of publication:
**11.03.2026 Bulletin 2026/11**

(21) Application number: **24918220.5**

(22) Date of filing: **21.11.2024**

(51) International Patent Classification (IPC):
*G06F 30/23* (2020.01)

(86) International application number:
**PCT/CN2024/133389**

(87) International publication number:
**WO 2025/152611 (24.07.2025 Gazette 2025/30)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **18.01.2024 CN 202410073794**

(71) Applicant: **Hefei Gotion High-Tech Power Energy Co., Ltd.**
**Hefei, Anhui 230012 (CN)**

(72) Inventors:
• **ZHANG, Yunlong**
**Hefei, Anhui 230012 (CN)**

• **ZHU, Chunlin**
**Hefei, Anhui 230012 (CN)**
• **SHI, Zheng**
**Hefei, Anhui 230012 (CN)**
• **CHEN, Haozhe**
**Hefei, Anhui 230012 (CN)**
• **JIAO, Yi**
**Hefei, Anhui 230012 (CN)**
• **ZHANG, Xu**
**Hefei, Anhui 230012 (CN)**

(74) Representative: **karo IP**
**Patentanwälte PartG mbB**
**Steinstraße 16-18**
**40212 Düsseldorf (DE)**

(54) **METHOD FOR ESTABLISHING POINT-RING LASER WELDING HEAT SOURCE MODEL, SYSTEM AND MEDIUM**

(57) The present invention discloses a method for establishing a heat source model of spot-ring laser welding, a system, and a medium, and belongs to the field of laser welding. Spot-ring laser includes spot laser of a central laser volume heat source and ring laser of an annular laser surface heat source. The former is used to implement a weld with a high aspect ratio for a to-be-welded material, and the latter is used to implement auxiliary heating and slow cooling for the to-be-welded material. Through the method for establishing a heat source model of spot-ring laser welding provided in the present invention, a laser power ratio of the spot laser and the ring laser in a model can be adjusted at any ratio, defocus amounts of the spot laser and the ring laser can be independently controlled, and spot parameters, such as a spot laser spot diameter and a ring laser spot diameter, can be used to effectively express heat flux density distribution. The method for establishing a heat source model provides a strong reference basis for accurately simulating a temperature field, a stress field, and a deformation field of spot-ring laser welding. In combination with regulation of a welding path, the present invention can be widely applied to the field of finite element simulation of linear welding or swing welding with spot-ring laser.

FIG. 1

EP 4 708 114 A1

Description

## TECHNICAL FIELD

The present invention relates to the field of laser welding, specifically, to the field of finite element simulation of laser welding, and in particular, to a method for establishing point-ring laser welding heat source model, system and medium.

## BACKGROUND

The laser welding technology is widely applied to fields such as new energy, airspace, and electronic devices, has advantages such as high precision, high efficiency, and a high automation degree, and is an advanced welding technology. With the development of automation technologies, the swing welding technology is gradually introduced into the field of laser welding. The swing motion of a laser beam is controlled by using a swing head and a galvanometer head. Such automated swing laser welding can improve stability, consistency, and precision of welding, and reduce human errors. The swing laser welding effectively resolves the problem of excessively high requirements on assembly precision in conventional narrow-gap laser welding. In recent years, as laser systems are constantly upgraded and improved, the ring-core adjustable laser welding technology is introduced to the laser welding market. The ring-core adjustable laser produced by Trumpf is the most representative. Ring-core adjustable laser, also referred to as spot-ring laser, is a brand new laser beam mode. The fiber of a conventional laser outputs only a single-spot light source, while spot-ring laser output by a ring-core adjustable laser is in a spot-ring laser beam mode formed by adding an annular light source outside a conventional spot light source. During welding, front-end ring laser in a welding direction preheats a metal material, and rear-end ring laser in the welding direction slows down cooling on the metal material. As been pointed out in related reports, compared with conventional laser welding, the spot-ring laser welding significantly reduces spattering. However, welding characteristics, a spot-ring energy ratio, process parameter matching, and a spatter suppression mechanism of spot-ring laser are still not clearly concluded. Energy distribution of spot-ring laser is different from that of conventional laser. There is a correlation between welding spatter and dynamic behavior of a keyhole during welding. When the correlation is analyzed from the perspective of energy distribution, flux behavior and heat transfer characteristics of a weld pool of a metal material during welding may be simulated through finite element simulation. However, establishing a welding heat source model is the first step of performing finite element simulation of laser welding, and it provides an energy distribution form of a welding heat source. Currently, there is no good model to represent a spot-ring laser welding heat source, especially, a spot-ring laser swing welding heat source. Therefore, it is necessary to provide a heat source model of spot-ring laser welding.

## SUMMARY

### 1. Technical problem to be solved

An objective of the present invention is to resolve the problem of difficulty in establishing a heat source model of spot-ring laser welding for a ring-core adjustable laser, to provide a method for establishing a heat source model of spot-ring laser welding, a system, and a medium.

### 2. Technical solution

The objective of the present invention can be achieved through the following technical solutions.

According to a first aspect of the present invention, a method for establishing a heat source model of spot-ring laser welding is provided, including the following steps:

establishing a heat source model of spot-ring laser welding, where spot-ring laser includes spot laser of a central laser volume heat source and ring laser of an annular laser surface heat source, and control equations of a spot-ring laser welding heat source are shown in formulas (1) and (2):

$$q_{\text{Total}}(x, y, z) = \frac{3\alpha Q \bullet u(z)}{\pi r_v^2 H} \exp\left(\frac{-3\beta(x^2 + y^2)}{r_v^2}\right)$$

$$+ \frac{(1-\alpha)Q}{\pi(r_2^2 - r_1^2)} \exp\left(\frac{-12\gamma(\sqrt{x^2 + y^2} - r_s)^2}{(r_2 - r_1)^2}\right), \tag{1}$$

$$r_v < r_1 < r_s < r_2$$

$$u(z) = \begin{cases} 1 & , -H \le z \le 0 \\ 0 & , z > 0, \text{ or } z < -H \end{cases} \tag{2}$$

in the formulas (1) and (2), $q_{\text{Total}}(x, y, z)$ represents a total heat flux density function of the spot-ring laser welding heat source, $(x, y, z)$ represents coordinates in a global coordinate system, $\alpha$ represents a laser power distribution coefficient and refers to a power ratio of the spot laser to the spot-ring laser, where a value range of the laser power distribution coefficient $\alpha$ is [0, 1], where $\alpha=0$ represents that a laser heat source is the ring laser, $0<\alpha<1$ represents that the laser heat source is the spot-ring laser, and $\alpha=1.0$ represents that the laser heat source is the spot laser, $Q$ represents a total input laser power, $\beta$ represents a heat flux concentration coefficient of central laser, $\gamma$ represents a heat flux concentration coefficient of annular laser, H represents an effective heating depth of the volume heat source and existence of a heat source heating effect when a heat source depth falls within a range of [-H, 0], and spot parameters include $r_v$, $r_s$, $r_l$, and $r_2$, where $r_v$ represents an effective heating radius of the central laser volume heat source, and the subscript v represents the volume heat source; $r_s$ represents a position at which a heat flux density of the annular laser surface heat source is the maximum, and the subscript s represents the surface heat source; and an effective heating range of the annular laser surface heat source is [$r_1$, $r_2$];
establishing a welding path equation, where the welding path equation is one or more of a linear welding control equation, a circular swing welding control equation, a sinusoidal swing welding control equation, and a linear swing welding control equation; and
obtaining a movement equation of the heat source model of spot-ring laser welding: selecting a required welding path form, and substituting the welding control equation corresponding to the welding path form into the control equation (1) of the welding heat source, to obtain a movement equation of the heat source model of spot-ring laser welding.

In some embodiments, a language code program of the heat source model of spot-ring laser welding in the formulas (1) and (2) is written through language compiler software, to generate data and an image of the welding heat source, to facilitate subsequent optimization. A heat flux density peak of the heat source is generated in the image, and a value range of the peak is from $10^6$ to $10^7$ W/cm$^2$.

Specifically, in the step of establishing a welding path equation:

the linear welding control equation is established as shown in formula (3):

$$\begin{cases} x = x_0 + vt \\ y = y_0 \\ z = z_0 \end{cases} \tag{3}$$

in the formula (3), (x, y, z) represents coordinates in the global coordinate system, $(x_0, y_0, z_0)$ represents coordinates of a welding starting position, $v$ represents a welding speed, and $t$ represents a welding time; and a welding track is a straight line.

The circular swing welding control equation is established as shown in formula (4):

$$\begin{cases} x = x_0 + vt + r \bullet \left[ 1 - \cos\left(\frac{2\pi}{T} t\right) \right] \\ y = y_0 + r \bullet \sin\left(\frac{2\pi}{T} t\right) \\ z = z_0 \end{cases} \qquad (4)$$

in the formula (4), $(x, y, z)$ represents coordinates in the global coordinate system, $(x_0, y_0, z_0)$ represents coordinates of a welding starting position, $v$ represents a welding speed, $t$ represents a welding time, $r$ represents a radius of a swing circle, and $T$ represents a swing cycle of the swing circle; and a welding track is a spiral line.

The sinusoidal swing welding control equation is established as shown in formula (5):

$$\begin{cases} x = x_0 + vt \\ y = y_0 + r \bullet \sin\left(\frac{2\pi}{T} t\right) \\ z = z_0 \end{cases} \qquad (5)$$

in the formula (5), $(x, y, z)$ represents coordinates in the global coordinate system, $(x_0, y_0, z_0)$ represents coordinates of a welding starting position, $v$ represents a welding speed, $t$ represents a welding time, $r$ represents a swing amplitude of a sinusoidal swing, and $T$ represents a swing cycle of the sinusoidal swing; and a welding track is a sinusoidal line.

The linear swing welding control equation is established as shown in formulas (6) to (8):

$$\begin{cases} x = x_0 + vt \\ y = y(t) \\ z = z_0 \end{cases} \qquad (6)$$

$$\begin{cases} t_1 = \dfrac{2(n-1)r}{v_1} + \dfrac{2(n-1)r}{v_2} \\ t_2 = \dfrac{(2n-1)r}{v_1} + \dfrac{2(n-1)r}{v_2} \\ t_3 = \dfrac{(2n-1)r}{v_1} + \dfrac{2nr}{v_2} \\ t_4 = \dfrac{2nr}{v_1} + \dfrac{2nr}{v_2} \end{cases} \quad , n = 1,2\ldots \qquad (7)$$

$$y(t) = \begin{cases} y_0 + v_1(t - t_1) & , t_1 \leq t < t_2 \\ y_0 + r - v_2(t - t_2) & , t_2 \leq t < t_3 \\ y_0 - r + v_1(t - t_3) & , t_3 \leq t < t_4 \end{cases} \qquad (8)$$

in the formulas (6) to (8), $(x, y, z)$ represents coordinates in the global coordinate system, $(x_0, y_0, z_0)$ represents coordinates of a welding starting position, $v$ represents a welding speed, $t$ represents a welding time, $r$ represents a swing amplitude of a linear swing, and proceeding and returning speeds within one swing cycle ( $\frac{2r}{v_1} + \frac{2r}{v_2}$ ) are respectively $v_1$ and $v_2$; and when $v_1 = -v_2$, a welding track is an isosceles triangle waveform.

The global coordinate system of the formula (3) to the formula (8) should match a coordinate system of a three-dimensional finite element mesh model. That is, coordinates $(x_0, y_0, z_0)$ in the movement equation of the heat source model of spot-ring laser welding are a welding starting position of the three-dimensional finite element mesh model, and a relative displacement between $z_0$ in the formula (3) to the formula (6) and a surface of a workpiece represents a defocus amount.

Further, a required welding path form is selected, and the formulas (3), (4), and (5) or the formulas (6) to (8) are selected to substituted into the formula (1), to obtain a movement equation of the heat source model of spot-ring laser welding.

In some embodiments, a language code program of a moving heat source model of spot-ring laser welding is written based on language compiler software, to help simulation software to subsequently call a subroutine.

Further, for the method for establishing a heat source model of spot-ring laser welding, a three-dimensional partial differential equation for solid thermal conduction with a heat source and a transient temperature field needs to be established and solved in finite element calculation software, to perform a thermal coupling operation. The three-dimensional partial differential equation for solid thermal conduction with a heat source and a transient temperature field is shown in formula (9):

$$\frac{\partial(\rho c_p T)}{\partial t} = k\left(\frac{\partial^2 T}{\partial x^2} + \frac{\partial^2 T}{\partial y^2} + \frac{\partial^2 T}{\partial z^2}\right) + q_{\text{Total}}(x, y, z) \qquad (9)$$

in the formula (9), $T$ represents a transient temperature of a material, $t$ represents a welding and cooling time, $k$ represents a thermal conductivity coefficient of the material, $\rho$ represents a density of the material, $c_p$ represents a specific heat of the material, $q_{\text{Total}}(x, y, z)$ represents the total heat flux density function of the spot-ring laser welding heat source shown in the formula (1), and $(x, y, z)$ represents the coordinates in the global coordinate system.

The method for establishing a heat source model of spot-ring laser welding in the present invention is applicable to a weldable thin-plate metal material, including, but not limited to, common metal materials such as aluminum alloy, stainless steel, titanium alloy, and copper alloy.

According to a second aspect of the present invention, a spot-ring laser welding system is provided, including a spot-ring laser, a spot-ring laser fiber, a welding head, and a calculation unit, where

the spot-ring laser is configured to generate spot-ring laser, and emit the spot-ring laser into the welding head through the spot-ring laser fiber;
the welding head is equipped with a motor and a control unit, where the control unit controls the motor to swing according to a set swing track; and
the calculation unit includes a memory, a processor, and a communication bus, where the communication bus implements connection and communication between the processor and the memory, and the processor executes one or more programs stored in the memory; and the calculation unit is configured to perform finite element simulation calculation and process optimization, and feed back a simulation result to the control unit, to set a swing track and a swing parameter, to run the method for establishing a heat source model of spot-ring laser welding according to the first aspect of the present invention.

According to a third aspect of the present invention, a computer-readable storage medium is provided, having one or more programs stored therein, where the one or more programs are executable by one or more processors, to run the method for establishing a heat source model of spot-ring laser welding according to the first aspect of the present invention.

### 3. Beneficial effects

Compared with the related art, advantages of the present invention are as follows: In the present invention, a brand new heat source model of spot-ring laser welding is established, so that energy distribution of a spot-ring beam mode can be well simulated. By adjusting a laser power ratio parameter, input powers of spot laser and ring laser can be adjusted at any ratio. By changing a welding starting position $(x_0, y_0, z_0)$, defocus amounts of spot laser and ring laser can be independently controlled. By combining spot parameters such as an effective heating radius of a central laser volume heat source and an effective heating range of an annular laser surface heat source, heat flux density distribution of spot-ring laser can be effectively expressed. The method for establishing a heat source model provides a strong reference basis for accurately simulating a temperature field, a stress field, and a deformation field of spot-ring laser welding. In combination with regulation of a welding path, the present invention can be widely applied to the field of finite element simulation of linear welding or swing welding with spot-ring laser.

### BRIEF DESCRIPTION OF THE DRAWINGS

FIG. 1 is a schematic diagram of establishing a heat source model of spot-ring laser welding according to the present invention;

FIG. 2 shows a heat source model of ring laser welding compiled based on a MATLAB language according to an embodiment of the present invention;

FIG. 3 shows a heat source model of spot-ring laser welding compiled based on a MATLAB language according to an embodiment of the present invention;

FIG. 4 shows a heat source model of spot laser welding compiled based on a MATLAB language according to an embodiment of the present invention;

FIG. 5 shows a longitudinal sectional morphology of a welding temperature field obtained by a heat source model of ring laser welding according to an embodiment of the present invention;

FIG. 6 shows a longitudinal sectional morphology of a welding temperature field obtained by a heat source model of spot-ring laser welding according to an embodiment of the present invention;

FIG. 7 shows a longitudinal sectional morphology of a welding temperature field obtained by a heat source model of spot laser welding according to an embodiment of the present invention;

FIG. 8 shows a cross-sectional morphology of a welding temperature field obtained by a heat source model of ring laser welding according to an embodiment of the present invention;

FIG. 9 shows a cross-sectional morphology of a welding temperature field obtained by a heat source model of spot-ring laser welding according to an embodiment of the present invention;

FIG. 10 shows a cross-sectional morphology of a welding temperature field obtained by a heat source model of spot laser welding according to an embodiment of the present invention;

FIG. 11 shows a weld surface morphology of a welding temperature field obtained by using a heat source model of circular swing spot laser according to an embodiment of the present invention;

FIG. 12 shows a weld surface morphology of a welding temperature field obtained by using a heat source model of sinusoidal swing spot laser according to an embodiment of the present invention; and

FIG. 13 shows a weld surface morphology of a welding temperature field obtained by using a heat source model of linear swing spot laser according to an embodiment of the present invention.

## DETAILED DESCRIPTION

The present invention is described below in detail with reference to the accompanying drawings of the specification and specific embodiments.

**Embodiment 1**

The technical solutions in the embodiments of the present invention are described below in detail with reference to the accompanying drawings and specific implementations. Apparently, the described embodiments are merely some of rather than all of the embodiments of the present invention. All other embodiments obtained by a person of ordinary skill in the art based on the embodiments of the present invention without creative efforts shall fall within the protection scope of the present invention.

The present invention provides a method for establishing a heat source model of spot-ring laser welding, including the following steps:

A heat source model of spot-ring laser welding is established.

Spot-ring laser includes spot laser of a central laser volume heat source and ring laser of an annular laser surface heat source, and control equations of a spot-ring laser welding heat source are shown in formulas (1) and (2):

$$q_{\text{Total}}(x, y, z) = \frac{3\alpha Q \bullet u(z)}{\pi r_v^2 H} \exp\left(\frac{-3\beta(x^2 + y^2)}{r_v^2}\right)$$

$$+ \frac{(1-\alpha)Q}{\pi(r_2^2 - r_1^2)} \exp\left(\frac{-12\gamma(\sqrt{x^2 + y^2} - r_s)^2}{(r_2 - r_1)^2}\right), \qquad (1)$$

$$r_v < r_1 < r_s < r_2$$

$$u(z) = \begin{cases} 1 & , -H \leq z \leq 0 \\ 0 & , z > 0, \text{ or } z < -H \end{cases} \qquad (2)$$

in the formulas (1) and (2), $q_{\text{Total}}(x, y, z)$ represents a total heat flux density function of the spot-ring laser welding heat source, $(x, y, z)$ represents coordinates in a global coordinate system, $\alpha$ represents a laser power distribution coefficient and refers to a power ratio of the spot laser to the spot-ring laser, $Q$ represents a total input laser power, $\beta$ represents a heat flux concentration coefficient of central laser, $\gamma$ represents a heat flux concentration coefficient of annular laser, $H$ represents an effective heating depth of the volume heat source and existence of a heat source heating effect when a heat source depth falls within a range of $[-H, 0]$, and spot parameters include $r_v$, $r_s$, $r_1$, and $r_2$, where $r_v$ represents an effective heating radius of the central laser volume heat source; $r_s$ represents a position at which a heat flux density of the annular laser surface heat source is the maximum; and an effective heating range of the annular laser surface heat source is $[r_1, r_2]$.

A MATLAB language code program of the heat source model of spot-ring laser welding in the formulas (1) and (2) is written through MATLAB language compiler software, to generate data and an image of the welding heat source, to facilitate subsequent optimization. A same function may also be implemented by using another language, which is not limited to MATLAB.

A welding path equation is established.

The linear welding control equation is established as shown in formula (3):

$$\begin{cases} x = x_0 + vt \\ y = y_0 \\ z = z_0 \end{cases} \qquad (3)$$

in the formula (3), (x, y, z) represents coordinates in the global coordinate system, $(x_0, y_0, z_0)$ represents coordinates of a welding starting position, $v$ represents a welding speed, and $t$ represents a welding time; and a welding track is a straight line.

The circular swing welding control equation is established as shown in formula (4):

$$\begin{cases} x = x_0 + vt + r \bullet \left[ 1 - \cos\left(\frac{2\pi}{T} t\right) \right] \\ y = y_0 + r \bullet \sin\left(\frac{2\pi}{T} t\right) \\ z = z_0 \end{cases} \qquad (4)$$

in the formula (4), (x, y, z) represents coordinates in the global coordinate system, $(x_0, y_0, z_0)$ represents coordinates of a welding starting position, $v$ represents a welding speed, $t$ represents a welding time, $r$ represents a radius of a swing circle, and $T$ represents a swing cycle of the swing circle; and a welding track is a spiral line.

The sinusoidal swing welding control equation is established as shown in formula (5):

$$\begin{cases} x = x_0 + vt \\ y = y_0 + r \bullet \sin\left(\frac{2\pi}{T} t\right) \\ z = z_0 \end{cases} \qquad (5)$$

in the formula (5), (x, y, z) represents coordinates in the global coordinate system, $(x_0, y_0, z_0)$ represents coordinates of a welding starting position, v represents a welding speed, $t$ represents a welding time, $r$ represents a swing amplitude of a sinusoidal swing, and $T$ represents a swing cycle of the sinusoidal swing; and a welding track is a sinusoidal line.

The linear swing welding control equation is established as shown in formulas (6) to (8):

$$\begin{cases} x = x_0 + vt \\ y = y(t) \\ z = z_0 \end{cases} \qquad (6)$$

$$\begin{cases} t_1 = \dfrac{2(n-1)r}{v_1} + \dfrac{2(n-1)r}{v_2} \\ t_2 = \dfrac{(2n-1)r}{v_1} + \dfrac{2(n-1)r}{v_2} \\ t_3 = \dfrac{(2n-1)r}{v_1} + \dfrac{2nr}{v_2} \\ t_4 = \dfrac{2nr}{v_1} + \dfrac{2nr}{v_2} \end{cases}, \quad n = 1,2\ldots \qquad (7)$$

$$y(t) = \begin{cases} y_0 + v_1(t - t_1) & , t_1 \le t < t_2 \\ y_0 + r - v_2(t - t_2) & , t_2 \le t < t_3 \\ y_0 - r + v_1(t - t_3) & , t_3 \le t < t_4 \end{cases} \qquad (8)$$

in the formulas (6) to (8), $(x, y, z)$ represents coordinates in the global coordinate system, $(x_0, y_0, z_0)$ represents coordinates of a welding starting position, $v$ represents a welding speed, $t$ represents a welding time, $r$ represents a swing amplitude of a linear swing, and proceeding and returning speeds within one swing cycle ( $\dfrac{2r}{v_1} + \dfrac{2r}{v_2}$ ) are respectively $v_1$ and $v_2$; and when $v_1 = -v_2$, a welding track is an isosceles triangle waveform.

A required welding path form is selected, and the formulas (3), (4), and (5) or the formulas (6) to (8) are selected to substituted into the formula (1), to obtain a movement equation of the heat source model of spot-ring laser welding. In this embodiment, a FORTRAN language code program of a moving heat source model of spot-ring laser welding is written based on FORTRAN language compiler software, to help simulation software to subsequently call a subroutine. Similarly, a language in which a related program is written and called is not limited.

The global coordinate system of the formula (3) to the formula (8) should match a coordinate system of a three-dimensional finite element mesh model. That is, coordinates $(x_0, y_0, z_0)$ in the movement equation of the heat source model of spot-ring laser welding are a welding starting position of the three-dimensional finite element mesh model, and a relative displacement between $z_0$ in the formula (3) to the formula (6) and a z-axis coordinate of the three-dimensional finite element mesh model represents a defocus amount.

For the establishing method, a three-dimensional partial differential equation for solid thermal conduction with a heat source and a transient temperature field needs to be established and solved in finite element calculation software, to perform a thermal coupling operation. The three-dimensional partial differential equation for solid thermal conduction with a heat source and a transient temperature field is shown in formula (9):

$$\frac{\partial(\rho c_p T)}{\partial t} = k\left( \frac{\partial^2 T}{\partial x^2} + \frac{\partial^2 T}{\partial y^2} + \frac{\partial^2 T}{\partial z^2} \right) + q_{\text{Total}}(x, y, z) \qquad (9)$$

in the formula (9), $T$ represents a transient temperature of a material, $t$ represents a welding and cooling time, $k$ represents a thermal conductivity coefficient of the material, $\rho$ represents a density of the material, $c_p$ represents a specific heat of the material, $q_{\text{Total}}(x, y, z)$ represents the total heat flux density function of the spot-ring laser welding heat source shown in the formula (1), and $(x, y, z)$ represents the coordinates in the global coordinate system.

The method for establishing a heat source model of spot-ring laser welding in the present invention is applicable to a weldable thin-plate metal material, including, but not limited to, common metal materials such as aluminum alloy, stainless steel, titanium alloy, and copper alloy.

This process is described below in detail by using specific embodiments.

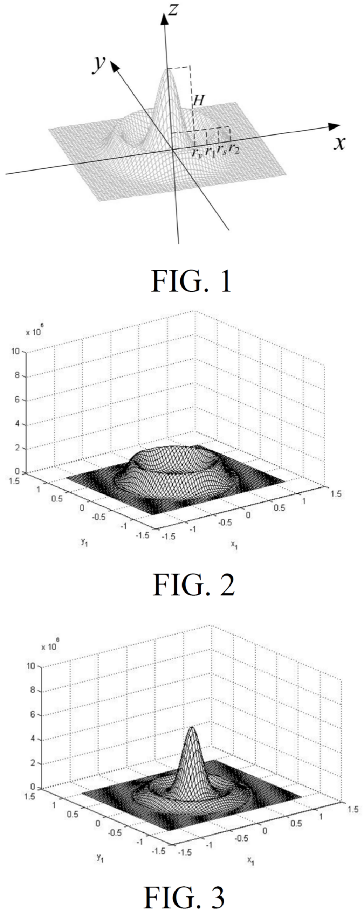

FIG. 1 is a schematic diagram of establishing a heat source model of spot-ring laser welding according to the present invention. According to FIG. 1, in formula (1) and formula (2), variable parameters are set as follows: an effective

heating radius $r_v$ of a central laser volume heat source is 0.3 mm, an effective heating range of an annular laser surface heat source is $r_1$=0.4 mm and $r_2$=0.8 mm, a position $r_s$ at which a heat flux density of the annular laser surface heat source is the maximum is 0.6 mm, and a total laser power $Q$ is 2000 W.

FIG. 2 to FIG. 4 respectively show heat source models of ring laser welding, spot-ring laser welding, and spot laser welding compiled based on a MATLAB language according to an embodiment of the present invention. A laser power distribution coefficient $\alpha$ is 0, 0.6, and 1.0 respectively, $\alpha$=0 represents that a laser heat source is the ring laser, $\alpha$=0.6 represents that a laser heat source is the spot-ring laser, and $\alpha$=1.0 represents that a laser heat source is the spot laser. A MATLAB language code program of the heat source model of spot-ring laser welding in the formulas (1) and (2) is written through MATLAB language compiler software, to generate data and an image of the welding heat source, as shown in FIG. 2 to FIG. 4.

A welding path equation is established. First, a linear welding control equation shown in formula (3) is selected. A FORTRAN language code program of a moving heat source model of spot-ring laser welding is written based on FORTRAN language compiler software, where a laser power distribution coefficient $\alpha$ is 0, 0.6, and 1.0 respectively, $\alpha$=0 represents that a laser heat source is the ring laser, $\alpha$=0.6 represents that a laser heat source is the spot-ring laser, and $\alpha$=1.0 represents that a laser heat source is the spot laser. The welding starting position is (0, 2, 0), and the welding speed is 200 mm/s.

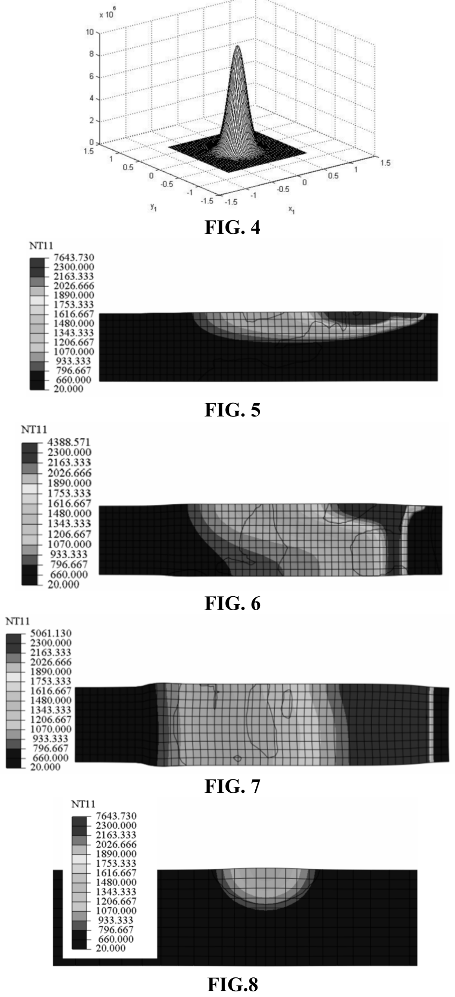

A three-dimensional geometric model is established by using ABAQUS finite element simulation software. Geometric dimensions of an aluminum alloy workpiece are 20 mm×10 mm×2 mm, and meshing is performed. A minimum mesh dimension is 0.2 mm, a maximum mesh dimension is 1.0 mm, a total quantity of meshes is 20000, and a total quantity of nodes is 230001. An eight-node thermal coupling hexahedral element is selected as a mesh type, which is suitable for thermal coupling calculation. Material properties and boundary conditions are established, and an initial temperature of the workpiece and an ambient temperature are both 20°C, which are submitted to finite element software for calculation and obtaining an analysis result. FIG. 5 to FIG. 7 respectively show longitudinal sectional morphologies of welding temperature fields obtained by a heat source model of ring laser welding, a heat source model of spot-ring laser welding, and a heat source model of spot laser welding according to an embodiment of the present invention. FIG. 8 to FIG. 10 respectively show cross-sectional morphologies of welding temperature fields obtained by a heat source model of ring laser welding, a heat source model of spot-ring laser welding, and a heat source model of spot laser welding according to an embodiment of the present invention. A part above 2300°C is a keyhole during welding. A part above 660°C is a liquid weld pool, and becomes a weld after cooling. Similarly, a type of finite element software used for modeling, meshing, and simulation calculation is not limited.

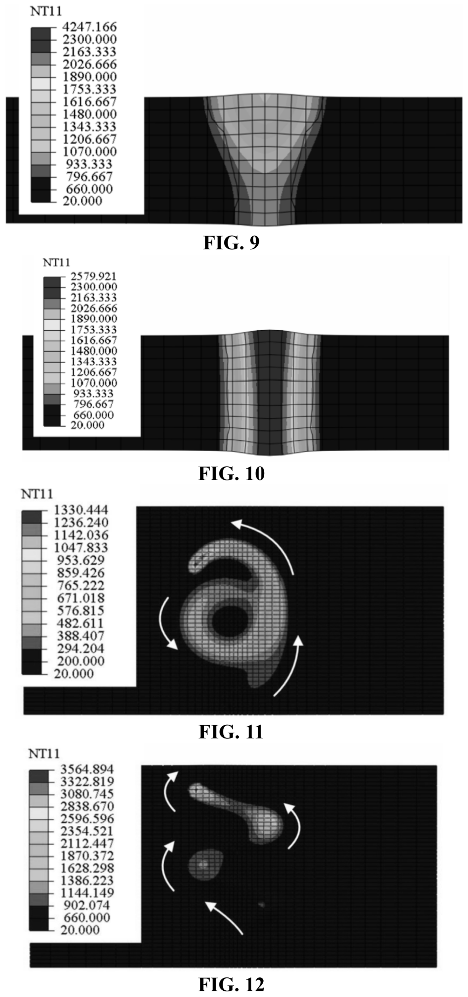

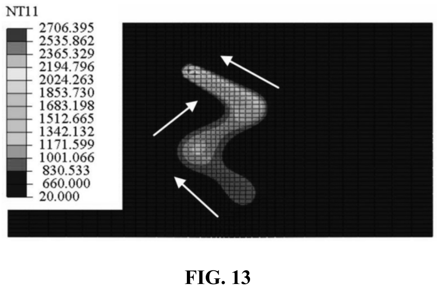

Circular, sinusoidal, and linear swing welding control equations shown in formulas (4), (5), and (6) to (8) are respectively selected, to finally obtain weld surface morphologies of welding temperature fields in different swing forms. FIG. 11 shows a weld surface morphology of a welding temperature field obtained by using a heat source model of circular swing spot laser according to an embodiment of the present invention, where a laser power distribution coefficient $\alpha$ is 1.0, a swing circle radius $r$ is 2 mm, and a swing cycle $T$ is 0.01s. FIG. 12 shows a weld surface morphology of a welding temperature field obtained by using a heat source model of sinusoidal swing spot laser according to an embodiment of the present invention, where a laser power distribution coefficient $\alpha$ is 1.0, a swing amplitude $r$ is 2 mm, and a swing cycle $T$ is 0.02s. FIG. 13 shows a weld surface morphology of a welding temperature field obtained by using a heat source model of linear swing spot laser according to an embodiment of the present invention, where a laser power distribution coefficient $\alpha$ is 1.0, a swing amplitude $r$ is 2 mm, and proceeding and returning speeds are $v_1$=400 mm/s and $v_2$=-400 mm/s. It may be understood that, although spot-ring laser (0<$\alpha$<1) is not used in a swing welding model in this embodiment of the present invention because a weld pool width is smaller when using spot laser, making a welding track directly visible, and a weld pool width is larger when using spot-ring laser, which may cover a welding track and make it difficult to distinguish the welding track, it does not affect use of spot-ring laser in heat source models of circular, sinusoidal, and linear swing welding. Values corresponding to different color icons in an image represent temperature values, in unit of degree Celsius.

By establishing a heat source model of spot-ring laser welding in the present invention, energy distribution of a spot-ring beam mode can be well simulated, a laser power ratio of the spot laser and the ring laser in a model can be adjusted at any ratio, defocus amounts of the spot laser and the ring laser can be independently controlled, and spot parameters, such as a spot laser spot diameter and a ring laser spot diameter, can be used to effectively express heat flux density distribution. The method for establishing a heat source model provides a strong reference basis for accurately simulating a temperature field, a stress field, and a deformation field of spot-ring laser welding. In combination with regulation of a welding path, the present invention can be widely applied to the field of finite element simulation of linear welding or swing welding with spot-ring laser.

**Embodiment 2**

According to a second aspect of the present invention, a spot-ring laser welding system is provided, including a spot-ring laser, a spot-ring laser fiber, a welding head, and a calculation unit. The spot-ring laser is configured to generate spot-ring laser, and emit the spot-ring laser into the welding head through the spot-ring laser fiber. The welding head is equipped with a motor and a control unit, where the control unit controls the motor to swing according to a set swing track. The calculation unit includes a memory, a processor, and a communication bus. The communication bus implements connection and communication between the processor and the memory, and the processor executes one or more programs stored in the memory. The calculation unit is configured to perform finite element simulation calculation and process optimization, and feed back a simulation result to the control unit, to set a swing track and a swing parameter, to implement the steps of the method for establishing a heat source model of spot-ring laser welding in Embodiment 1.

**Embodiment 3**

According to a third aspect of the present invention, an embodiment of this application further provides a computer-readable storage medium. The computer-readable storage medium has one or more programs stored therein. The one or more programs may be executed by one or more processors, to perform any step of the method for establishing a heat source model of spot-ring laser welding in Embodiment 1.

The present invention and implementations thereof have been described illustratively above; however, the description is non-limiting. The present invention can be implemented in other specific forms without departing from the spirit or basic characteristics of the present invention. The content shown in the accompanying drawings is merely one of the implementations of the present invention, and the actual structure is not limited thereto. Any reference sign in the claims should not be construed as limiting the claims. Therefore, similar structures and embodiments designed by a person of ordinary skill in the art as inspired by the disclosure herein without departing from the spirit of the present invention and without creative efforts shall fall within the protection scope of the present invention. In addition, the word "include" does not exclude other elements or steps, and the word "a/an" before an element does not exclude "a plurality" of the elements. A plurality of elements stated in the product claims can also be implemented by one element through software or hardware. The words, such as "first" and "second", are only used to indicate names, and do not indicate any particular order.

**Claims**

1.    A method for establishing a heat source model of spot-ring laser welding, wherein spot-ring laser comprises spot laser of a central laser volume heat source and ring laser of an annular laser surface heat source, and control equations of a spot-ring laser welding heat source are shown in formulas (1) and (2):

$$q_{\text{Total}}(x,y,z) = \frac{3\alpha Q \bullet u(z)}{\pi r_v^2 H} \exp\left(\frac{-3\beta(x^2+y^2)}{r_v^2}\right)$$

$$+ \frac{(1-\alpha)Q}{\pi(r_2^2-r_1^2)} \exp\left(\frac{-12\gamma(\sqrt{x^2+y^2}-r_s)^2}{(r_2-r_1)^2}\right), \tag{1}$$

$$r_v < r_1 < r_s < r_2$$

$$u(z) = \begin{cases} 1 & , -H \leq z \leq 0 \\ 0 & , z > 0, \text{ or } z < -H \end{cases} \tag{2}$$

in the formulas (1) and (2), $q_{\text{Total}}(x, y, z)$ represents a total heat flux density function of the spot-ring laser welding heat source, $(x, y, z)$ represents coordinates in a global coordinate system, $\alpha$ represents a laser power distribution coefficient and refers to a power ratio of the spot laser to the spot-ring laser, $Q$ represents a total input laser power, $\beta$ represents a heat flux concentration coefficient of central laser, $\gamma$ represents a heat flux concentration coefficient of annular laser, $H$ represents an effective heating depth of the volume heat source and existence of a heat source heating effect when a heat source depth falls within a range of [-$H$, 0], and spot parameters comprise $r_v$, $r_s$, $r_1$, and $r_2$, wherein $r_v$ represents an effective heating radius of the central laser volume heat source, and the subscript v represents the volume heat source; $r_s$ represents a position at which a heat flux density of the annular laser surface heat source is the maximum, and the subscript s represents the surface heat source; and an effective heating range of the annular laser surface heat source is $[r_1, r_2]$.

2. The method for establishing a heat source model of spot-ring laser welding according to claim 1, comprising the following steps:

establishing a heat source model of spot-ring laser welding, wherein the control equations of the spot-ring laser welding heat source are shown in the formulas (1) and (2):

establishing a welding path equation, wherein the welding path equation is one or more of a linear welding control equation, a circular swing welding control equation, a sinusoidal swing welding control equation, and a linear swing welding control equation; and

selecting a required welding path form, and substituting the welding control equation corresponding to the welding path form into the control equation (1) of the welding heat source, to obtain a movement equation of the heat source model of spot-ring laser welding.

3. The method for establishing a heat source model of spot-ring laser welding according to claim 2, further comprising the following step: writing a code program of a moving heat source model of spot-ring laser welding according to the movement equation of the heat source model of spot-ring laser welding, to generate data and an image of the welding heat source, wherein the image comprises a heat flux density peak of the heat source.

4. The method for establishing a heat source model of spot-ring laser welding according to claim 3, wherein a range of the heat flux density peak of the heat source is from $10^6$ to $10^7$ W/cm$^2$.

5. The method for establishing a heat source model of spot-ring laser welding according to claim 4, wherein

the linear welding control equation is established as shown in formula (3):

$$\begin{cases} x = x_0 + vt \\ y = y_0 \\ z = z_0 \end{cases} \qquad (3)$$

in the formula (3), $(x, y, z)$ represents coordinates in the global coordinate system, $(x_0, y_0, z_0)$ represents coordinates of a welding starting position, $v$ represents a welding speed, and $t$ represents a welding time; and a welding track is a straight line.

6. The method for establishing a heat source model of spot-ring laser welding according to claim 4, wherein the circular swing welding control equation is established as shown in formula (4):

$$\begin{cases} x = x_0 + vt + r \bullet \left[ 1 - \cos\left(\frac{2\pi}{T} t\right) \right] \\ y = y_0 + r \bullet \sin\left(\frac{2\pi}{T} t\right) \\ z = z_0 \end{cases} \qquad (4)$$

in the formula (4), $(x, y, z)$ represents coordinates in the global coordinate system, $(x_0, y_0, z_0)$ represents coordinates of a welding starting position, $v$ represents a welding speed, $t$ represents a welding time, $r$ represents a radius of a swing circle, and $T$ represents a swing cycle of the swing circle; and a welding track is a spiral line.

7. The method for establishing a heat source model of spot-ring laser welding according to claim 4, wherein the sinusoidal swing welding control equation is established as shown in formula (5):

$$\begin{cases} x = x_0 + vt \\ y = y_0 + r \bullet \sin\left(\dfrac{2\pi}{T} t\right) \\ z = z_0 \end{cases} \tag{5}$$

in the formula (5), $(x, y, z)$ represents coordinates in the global coordinate system, $(x_0, y_0, z_0)$ represents coordinates of a welding starting position, $v$ represents a welding speed, $t$ represents a welding time, $r$ represents a swing amplitude of a sinusoidal swing, and $T$ represents a swing cycle of the sinusoidal swing; and a welding track is a sinusoidal line.

8. The method for establishing a heat source model of spot-ring laser welding according to claim 4, wherein the linear swing welding control equation is established as shown in formulas (6) to (8):

$$\begin{cases} x = x_0 + vt \\ y = y(t) \\ z = z_0 \end{cases} \tag{6}$$

$$\begin{cases} t_1 = \dfrac{2(n-1)r}{v_1} + \dfrac{2(n-1)r}{v_2} \\ t_2 = \dfrac{(2n-1)r}{v_1} + \dfrac{2(n-1)r}{v_2} \\ t_3 = \dfrac{(2n-1)r}{v_1} + \dfrac{2nr}{v_2} \\ t_4 = \dfrac{2nr}{v_1} + \dfrac{2nr}{v_2} \end{cases}, \ n = 1,2\ldots \tag{7}$$

$$y(t) = \begin{cases} y_0 + v_1(t - t_1) & , t_1 \le t < t_2 \\ y_0 + r - v_2(t - t_2) & , t_2 \le t < t_3 \\ y_0 - r + v_1(t - t_3) & , t_3 \le t < t_4 \end{cases} \tag{8}$$

in the formulas (6) to (8), $(x, y, z)$ represents coordinates in the global coordinate system, $(x_0, y_0, z_0)$ represents coordinates of a welding starting position, v represents a welding speed, $t$ represents a welding time, $r$ represents a swing amplitude of a linear swing, and proceeding and returning speeds within one swing cycle ( $\dfrac{2r}{v_1} + \dfrac{2r}{v_2}$ ) are respectively $v_1$ and $v_2$; and when $v_1 = -v_2$, a welding track is an isosceles triangle waveform.

9. The method for establishing a heat source model of spot-ring laser welding according to any one of claims 1 to 8, wherein a three-dimensional partial differential equation for solid thermal conduction with a heat source and a transient temperature field is further established to perform a thermal coupling operation, wherein the three-dimensional partial differential equation for solid thermal conduction with a heat source and a transient temperature field is shown in formula (9):

$$\frac{\partial(\rho c_p T)}{\partial t} = k\left(\frac{\partial^2 T}{\partial x^2} + \frac{\partial^2 T}{\partial y^2} + \frac{\partial^2 T}{\partial z^2}\right) + q_{\text{Total}}(x, y, z) \tag{9}$$

in the formula (9), $T$ represents a transient temperature of a material, $t$ represents a welding and cooling time, $k$ represents a thermal conductivity coefficient of the material, $\rho$ represents a density of the material, and $c_p$ represents a specific heat of the material.

10. A spot-ring laser welding system, comprising a spot-ring laser, a spot-ring laser fiber, a welding head, and a calculation unit, wherein

the spot-ring laser is configured to generate spot-ring laser, and emit the spot-ring laser into the welding head through the spot-ring laser fiber;

the welding head is equipped with a motor and a control unit, wherein the control unit controls the motor to swing according to a set swing track; and

the calculation unit comprises a memory, a processor, and a communication bus, wherein the communication bus implements connection and communication between the processor and the memory, and the processor executes one or more programs stored in the memory; and the calculation unit is configured to perform finite element simulation calculation and process optimization, and feed back a simulation result to the control unit, to set a swing track and a swing parameter, to run the method for establishing a heat source model of spot-ring laser welding according to any one of claims 1 to 9.

11. A computer-readable storage medium, having one or more programs stored therein, wherein the one or more programs are executable by one or more processors, to run the method for establishing a heat source model of spot-ring laser welding according to any one of claims 1 to 9.

FIG. 1

FIG. 2

FIG. 3

**FIG. 4**

**FIG. 5**

**FIG. 6**

**FIG. 7**

**FIG.8**

**FIG. 9**

**FIG. 10**

**FIG. 11**

**FIG. 12**

**FIG. 13**

## INTERNATIONAL SEARCH REPORT

| International application No. |
| --- |
| **PCT/CN2024/133389** |

**A. CLASSIFICATION OF SUBJECT MATTER**

G06F30/23(2020.01)i

According to International Patent Classification (IPC) or to both national classification and IPC

**B. FIELDS SEARCHED**

Minimum documentation searched (classification system followed by classification symbols)

IPC:G06F

Documentation searched other than minimum documentation to the extent that such documents are included in the fields searched

Electronic data base consulted during the international search (name of data base and, where practicable, search terms used)

CNABS; CNTXT; DWPI; VEN; USTXT; WOTXT; EPTXT; CNKI; IEEE; BING; 百度, BAIDU: 点光, 环光, 激光, 焊接, 热源, 模型, 功率, 密度, 加热半径, 深度, point light, ring light, laser, welding, heat source, model, power, density, heating radius, depth

**C. DOCUMENTS CONSIDERED TO BE RELEVANT**

| Category* | Citation of document, with indication, where appropriate, of the relevant passages | Relevant to claim No. |
| --- | --- | --- |
| PX | CN 117973120 A (HEFEI GOTION HIGH-TECH POWER ENERGY CO., LTD.) 03 May 2024 (2024-05-03) claims 1-11, and description, paragraphs 61-100 | 1-11 |
| A | CN 115639782 A (HUAIHUA UNIVERSITY) 24 January 2023 (2023-01-24) description, paragraphs 56-109 | 1-11 |
| A | CN 111914383 A (NANJING UNIVERSITY OF AERONAUTICS AND ASTRONAUTICS) 10 November 2020 (2020-11-10) description, paragraphs 5-22 | 1-11 |
| A | CN 115544683 A (XI'AN INSTITUTE OF SPACE RADIO TECHNOLOGY) 30 December 2022 (2022-12-30) entire document | 1-11 |
| A | WO 2022212164 A1 (NUTECH VENTURES) 06 October 2022 (2022-10-06) entire document | 1-11 |

☑ Further documents are listed in the continuation of Box C. ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" | document member of the same patent family |
| "P" | document published prior to the international filing date but later than the priority date claimed | | |

| Date of the actual completion of the international search | Date of mailing of the international search report |
| --- | --- |
| **25 February 2025** | **27 February 2025** |

| Name and mailing address of the ISA/CN | Authorized officer |
| --- | --- |
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**

International application No.

**PCT/CN2024/133389**

**C.    DOCUMENTS CONSIDERED TO BE RELEVANT**

| Category* | Citation of document, with indication, where appropriate, of the relevant passages | Relevant to claim No. |
|---|---|---|
| A | 王慧 等 (WANG, Hui et al.). "基于组合热源模型的激光深熔焊数值模拟 (Numerical Simulation of Laser Deep-penetration Welding Based on the Combined Heat Source Model)" 武汉理工大学学报 (Journal of Wuhan University of Technology), Vol. 36, No. 11, 30 November 2014 (2014-11-30), ISSN: 1671-4431, pages 39-45 | 1-11 |

Form PCT/ISA/210 (second sheet) (July 2022)

**INTERNATIONAL SEARCH REPORT**
Information on patent family members

International application No.

**PCT/CN2024/133389**

| Patent document cited in search report | | | Publication date (day/month/year) | Patent family member(s) | | | Publication date (day/month/year) |
|---|---|---|---|---|---|---|---|
| CN | 117973120 | A | 03 May 2024 | None | | | |
| CN | 115639782 | A | 24 January 2023 | None | | | |
| CN | 111914383 | A | 10 November 2020 | None | | | |
| CN | 115544683 | A | 30 December 2022 | None | | | |
| WO | 2022212164 | A1 | 06 October 2022 | US | 2024169115 | A1 | 23 May 2024 |

Form PCT/ISA/210 (patent family annex) (July 2022)